# FASCICULE DE BREVET EUROPEEN

(11) **EP 0 544 578 B1**
(45) Date de publication et mention de la délivrance du brevet: **25.06.1997**
(21) Numéro de dépôt: 92403152.9
(22) Date de dépôt: 24.11.1992
(51) Int. Cl.: G01N 33/543, G01N 33/577, G01N 33/538, G01N 33/553

(54) **Trousse pour le dénombrement rapide des granulocytes et procédé utilisant ladite trousse**
Satz zur schnellen Aufzählung von Granulocyten und Verfahren das diesen Satz verwendet
Kit for the rapid enumeration of granulocytes and method using said kit

(30) Priorité: 25.11.1991 FR 9114508
(43) Date de publication de la demande: 02.06.1993
(73) Titulaire: PASTEUR SANOFI DIAGNOSTICS, F-92430 Marnes La Coquette (FR)
(72) Inventeur: Carriere, Dominique, F-34160 Castries (FR)
(74) Mandataire: Gillard, Marie-Louise

(56) Documents cités:
- EP-A- 0 297 290
- EP-A- 0 311 492
- EP-A- 0 317 156
- WO-A-89/11102
- WO-A-90/15328

## Description

La présente invention a pour objet une trousse pour le dénombrement rapide des granulocytes et un procédé utilisant ladite trousse.

Les leucocytes polynucléaires, encore appelés granulocytes comprennent 3 sous-populations : les polynucléaires neutrophiles, basophiles et éosinophiles.

Les granulocytes, présents normalement dans le sang périphérique, peuvent être observés en pathologie infectieuse dans d'autres liquides biologiques tels que l'urine, le liquide céphalo-rachidien , le liquide pleural ou le liquide provenant d'un lavage broncho-alvéolaire.

Le nombre normal moyen de leucocytes polynucléaires du sang peut être modifié dans certaines circonstances. Une augmentation anormale du nombre de leucocytes polynucléaires sanguins peut être le signe d'une situation pathologique, par exemple l'hypergranulocytose provoquée par un agent infectieux au cours des hyperéosinophilies ou dans les leucémies myeloïdes. Inversement, une diminution anormale du nombre des leucocytes polynucléaires (granulopénie) peut être induite par des médicaments ou des traitements de chimiothérapie. Certains médicaments peuvent provoquer des modifications importantes du nombre de granulocytes ; ceci est décrit par exemple par P. Tarallo, J.F. Guelfi dans Examens de Laboratoires et Médicaments ; ed. G. Siest et al., Expansion Scientifique, 1985, 163-181. Les effets indésirés de ces médicaments doivent être évalués de façon systématique et régulière par le comptage du nombre de cellules sanguines (numération et formule sanguine).

Ainsi, un des examens pratiqués le plus souvent en hématologie est l'établissement de la formule sanguine. Pour les hématies et chacune des populations leucocytaires, on détermine les valeurs moyennes des numérations cellulaires et on les compare aux valeurs limites (inférieures et supérieures) considérées comme normales, déterminées par ailleurs.

A titre d'exemple, avant d'entreprendre un traitement de chimiothérapie et en cours de traitement, on effectue des numérations des granulocytes pour surveiller l'état du patient et adapter le traitement en conséquence. Dans les pratiques thérapeutiques modernes, la chimiothérapie est souvent administrée en hospitalisation de jour. Il est alors très utile pour le médecin de disposer d'une trousse pour le dénombrement rapide des granulocytes permettant d'obtenir facilement un résultat quasi immédiat.

A ce jour les numérations des cellules sanguines sont réalisées par microscopie ou à l'aide d'automates. On peut par exemple utiliser un compteur électronique de type Coulter Counter : Coultronics ® dans lequel la mesure est basée sur la variation de la résistivité du milieu dans lequel se trouvent les cellules. D'autres appareils, comme l'Hematolog D ® utilisent des mesures cytochimiques.

La connaissance des antigènes ou marqueurs de la surface cellulaire a fait d'énormes progrès avec la mise au point de l'hybridation lymphocytaire et la découverte des anticorps monoclonaux par Koehler et Milstein (Nature, 1975, 256, 495-497).

Par convention internationale, les marqueurs de surface des leucocytes humains ont été classés dans des groupes ou classes de différentiation (CD) définis lors du sous-comité IUIS-OMS, 1984, décrits dans le Bulletin de l'Organisation Mondiale de la Santé, 1984, 62 (5), 813-815 et régulièrement remis à jour.

La demande de brevet européen EP 297 290 décrit une méthode pour la détermination immunométrique d'un analyte dans un fluide biologique. Elle utilise (i) des particules magnétiquement sensibles sur lesquelles est immobilisé l'analyte à doser, (ii) un réactif marqué constitué d'un anticorps spécifique dirigé contre ledit analyte, marqué par un groupe chimique détectable et (iii) un dispositif de séparation et de détection de l'analyte marqué. Selon cette méthode, l'analyte marqué est "transporté" de la zone de séparation à la zone de détection par capillarité.

La demande de brevet européen 311 492 décrit une méthode immunométrique pour le dosage des antigènes de surface caractéristiques d'une population cellulaire. Elle utilise, d'une part un support solide sur lequel sont fixés des anticorps monoclonaux dirigés contre des antigènes de surface de la population cellulaire à doser et d'autre part une solution contenant d'autres anticorps monoclonaux marqués, spécifiques de la population cellulaire à doser. Par cette méthode, on réalise en une seule étape l'immobilisation spécifique de la population cellulaire à doser par immunocapture sur un support solide et la reconnaissance, par un anticorps marqué, d'un antigène de surface de ladite population.

Parmi les exemples d'utilisation décrits dans la demande EP 311 492, plusieurs concernent le dosage d'antigènes de surface portés par des cellules sanguines. On constate que le dosage proprement dit est toujours précédé d'une étape de séparation des cellules sanguines du sang total et l'exemple 5 étudie l'influence de la méthode de séparation choisie (centrifugation ou lyse des érythrocytes) sur les résultats du dosage. Dans le même exemple, on détermine également l'influence du temps d'incubation pour l'immunocapture et le marquage des cellules sur les résultats observés et l'on montre qu'un signal spécifique analytiquement exploitable est atteint après 10 minutes de contact. D'après les exemples décrits, on constate cependant que le temps d'incubation effectif n'est jamais inférieur à 20 minutes.

L'exemple 15 de la demande EP 311 492 décrit le dosage de l'antigène CD 5 des lymphocytes T humains en utilisant des billes magnétiques recouvertes d'anticorps anti-CD 2 comme support solide pour l'immunocapture. Dans cet exemple, les cellules mononucléées sont également séparées du sang total avant l'étape d'incubation pour l'immunocapture et le dosage. De plus, dans lesdits exemples, l'étape d'incubation dure 1 heure à elle seule.

L'exemple 11 décrit le dosage des antigènes CD15 portés sur les granulocytes humains. La détermination est effectuée sur des granulocytes provenant du sang total : dans un premier temps, les granulocytes sont séparés des globules rouges par mélange avec un milieu physiologique utilisé en perfusion (Plasmion®) puis, après 45 minutes de repos, on prélève la suspension de leucocytes à la surface du milieu liquide. Cette étape de séparation préalable dure plus d'une heure. Les granulocytes sont ensuite immobilisés dans les puits d'une plaque de microtitration par un anticorps monoclonal spécifique de l'antigène CD45. Cet antigène CD45 est présent sur la membrane cellulaire de tous les leucocytes. Pour le dosage, on utilise l'anticorps anti-CD15 marqué à la peroxydase.

Par ailleurs, dans les différents exemples décrits dans la demande EP 311 492, on constate l'importance, en nombre de manipulations et en temps d'exécution, de l'étape de lavage du support solide sur lequel est fixée la population cellulaire à doser.

Ainsi, malgré le souci de rapidité énoncé dans cette demande de brevet, la durée totale d'exécution du dosage est toujours longue : inférieure ou égale à 1 heure. En effet, les étapes suivantes sont nécessaires pour effectuer le dosage:
- préparation de l'échantillon à doser avec, le cas échéant, séparation des cellules sanguines du sang total ;
- incubation pendant au moins 10 minutes, de préférence davantage ;
- lavage du support solide ;
- dans le cas d'un marquage enzymatique, révélation de l'activité enzymatique (20 à 25 minutes avec la peroxydase, plus longtemps avec d'autres enzymes).

L'ensemble de ces opérations est réalisée par un homme du métier en un temps compris entre 45 et 60 minutes.

Enfin, l'expression des résultats nécessite un étalonnage en fonction de la méthode de mesure choisie, étalonnage réalisé avec une préparation cellulaire préalablement calibrée en antigènes par la méthode de cytofluorométrie quantitative.

Selon la présente invention , on a trouvé un système de dénombrement des granulocytes basé sur une mesure immunométrique qui satisfait aux critères des tests de diagnostic rapide, en particulier, la simplicité de mise en oeuvre, la rapidité d'exécution et la fiabilité des résultats :
- la mesure des granulocytes est effectuée en utilisant le même anticorps pour la capture et le marquage des cellules à doser et l'on mesure ainsi directement le nombre de cellules ;
- la mesure est réalisée sur un échantillon de sang total ou de liquide biologique, ce qui évite une étape préalable longue de séparation des leucocytes polynucléaires du sang complet ou du liquide biologique;
- l'utilisation de particules magnétiques appropriées et la méthode choisie pour la capture et le lavage desdites particules portant les cellules à mesurer permettent de simplifier et de raccourcir le temps de lavage ;
- la possibilité d'adjoindre très simplement un étalon interne peut conduire à une expression directe d'un résultat de mesure.

Selon la présente invention, l'anticorps utilisé pour la capture et le marquage des granulocytes, ci-après appelé anticorps spécifique, est un anticorps monoclonal dirigé contre un antigène de surface spécifique des granulocytes.

Ainsi ledit antigène est présent à la surface des granulocytes et non présent sur d'autres cellules ; de plus cet antigène est suffisamment abondant à la surface des granulocytes pour permettre à la fois leur capture et leur marquage par l'anticorps spécifique.

La présente invention a pour objet une trousse pour le dénombrement rapide des granulocytes du sang humain ou de tout autre liquide biologique humain, normal ou pathologique, comprenant comme composants :
a) un premier récipient contenant une quantité déterminée de réactif de dilution et de marquage des granulocytes contenant un anticorps spécifique marqué d'une façon usuelle ;
b) un deuxième récipient contenant des particules magnétiques sur lesquelles est fixé le même anticorps spécifique non marqué ;
c) une baguette, dite baguette de mesure, pourvue d'une partie terminale aimantée ;

Avantageusement, la trousse selon l'invention peut également contenir un ou plusieurs des éléments suivants :
d) un dispositif permettant de prélever une quantité déterminée de sang ou de liquide biologique;
e) un dispositif contenant du liquide de lavage ;
f) des moyens pour révéler le marqueur de l'anticorps spécifique, par exemple, dans le cas d'anticorps spécifique marqué par une sonde enzymatique, un ou plusieurs récipients contenant le révélateur de l'enzyme , à savoir une ou plusieurs solutions contenant le substrat de l'enzyme et, le cas échéant, un ou plusieurs réactifs nécessaires pour mesurer l'activité de l'enzyme ;
g) au moins une baguette, dite baguette de référence, ladite baguette étant recouverte sur sa partie terminale d'une quantité déterminée différente pour chacune, soit d'anticorps polyclonaux anti-espèce, ladite espèce étant l'espèce animale de laquelle provient l'anticorps spécifique, soit d'antigène spécifique.

Selon un autre mode de réalisation de l'invention, la trousse ne contient ni baguette de mesure, ni baguette de référence, mais son utilisation fait intervenir un barreau aimanté permettant, par application sur la surface externe du récipient contenant le réactif de capture et de marquage, d'immobiliser les particules magnétiques sur la surface interne dudit récipient.

Lorsque la trousse ne contient pas de baguette de référence, on peut y adjoindre différents échantillons de référence contenant chacun un nombre connu de granulocytes, mesuré par comptage à l'aide d'un appareil automatique.

Par "dispositif permettant de prélever une quantité déterminée de sang ou de liquide biologique", on entend une seringue de volume déterminé, équipée d'une aiguille, permettant de prélever du sang veineux ou un piqueur auquel on adjoint un tube capillaire calibré ou encore, tout dispositif approprié permettant de prélever un volume donné de sang ou de liquide biologique, à partir d'un échantillon existant.

Les particules magnétiques utilisées dans la trousse selon la présente invention ont des formes variables : sphériques ou non sphériques. Préférentiellement, leur plus grande dimension est comprise entre 0,1 et 20 µm. A titre d'exemple, on peut citer des particules magnétiques à enrobage de polystyrène, comme les particules Estapor ® , commercialisées par Rhône-Poulenc, dont les dimensions varient de 0,1 à 10 µm, la valeur moyenne étant 0,8 µm. On peut également citer des particules magnétiques composites multicouches, comme les particules Dynabeads ® , commercialisées par Dynal, dont la dimension est d'environ 2 µm.

On peut aussi utiliser des particules préparées selon le brevet européen 104 101 par polymérisation de dérivés acryliques, sous irradiation gamma, en présence d'un matériau magnétique préparé par une méthode connue, par exemple en utilisant le procédé décrit par M. Ronay dans American Chemical Society Symposium Serie, 1982, 200 Reprographic Technology, 553-576. La dimension de ces particules est comprise entre 1 et 10 µm.

De façon particulièrement préférentielle, les particules magnétiques utilisées dans la trousse selon l'invention ont une dimension comprise entre 0,5 et 4 µm.

La fixation de l'anticorps monoclonal sur les particules magnétiques, est réalisée par adsorption physique ou par liaison covalente. On peut utiliser un procédé proposé par les fournisseurs, lorsque les particules sont commercialisées, ou bien le procédé décrit dans le brevet européen 104 101.

Le récipient contenant les particules magnétiques est préparé selon un mode opératoire usuel ; les particules magnétiques sur lesquelles sont fixés les anticorps monoclonaux sont placées dans un tampon phosphate à pH voisin de 7, rendu isotonique par addition de chlorure de sodium et contenant des protéines comme la sérum albumine bovine ou le sérum de veau foetal. Une faible quantité de cette solution est placée dans le récipient, celui-ci est ensuite lyophilisé, bouché, puis stocké à basse température (inférieure à 5°C). Par faible quantité, on entend un volume environ 100 fois inférieur au volume du récipient.

L'anticorps spécifique marqué est obtenu de façon usuelle par l'une quelconque des méthodes de marquage bien connues de l'homme de l'art, telles que le marquage radioisotopique, le marquage enzymatique ou le marquage par un agent fluorescent.

Les granulocytes ont des dimensions d'environ 10 à 15 µm et sont de forme irrégulière ; ils portent à leur surface de nombreux marqueurs cellulaires ou antigènes. Certains d'entre eux, comme l'antigène CD45, sont présents sur tous les leucocytes, d'autres, comme le CD15, sont spécifiques des granulocytes.

Pour un mode de réalisation particulier du dosage immunométrique selon la présente invention, on a choisi l'antigène cellulaire CD15 pour la capture et le marquage des granulocytes. Cet antigène est très abondant à la surface des granulocytes.

Ainsi, la capture et le marquage des granulocytes, en vue de la détermination du nombre de ces cellules, sont effectués par un anticorps monoclonal anti-CD15 présentant une affinité pour l'antigène supérieure à 10⁸ M⁻¹.

Selon un mode de réalisation particulier de la présente invention, l'anticorps monoclonal utilisé est une immunoglobuline d'isotype IgM. Une telle molécule d'immunoglobuline possède 10 sites anticorps alors qu'une molécule d'immunoglobuline d'isotype IgG ne possède que 2 sites anticorps.

Par le grand nombre de leurs sites de liaison (5 fois plus élevé que celui d'une immunoglobine d'isotype IgG), les IgM anti-CD15 offrent une plus grande capacité à reconnaître les antigènes CD15 présents à la surface des granulocytes, donc à capturer lesdits granulocytes. De plus les IgM anti-CD15 peuvent porter davantage de molécules de traceur constituant la sonde enzymatique ou radioisotopique, ce qui leur confère une très forte capacité de marquage des antigènes CD15 des granulocytes.

L'anticorps SMY 15 a, commercialisé par BIOSYS est une immunoglobuline IgM qui convient tout particulièrement pour la réalisation du dosage selon l'invention.

On peut également utiliser l'anticorps anti-CD15, clone 80H5, commercialisé par Immunotech.

Par réactif de dilution et de marquage des granulocytes, on entend le réactif contenant l'anticorps anti-CD15 fluorescent ou marqué par une sonde enzymatique ou par une sonde radioisotopique, dilué dans un tampon salin de pH voisin de 7, par exemple un tampon phosphate salin, ledit tampon contenant des protéines comme la sérum albumine bovine ou du sérum de veau foetal.

Le réactif est utilisé de telle sorte que la concentration de l'anticorps anti-CD15 contenu dans ce réactif soit comprise entre 0,5 et 5 µg/ml.

Par liquide de lavage, on entend un liquide physiologique classique pour le lavage, tel qu'un tampon salin de pH voisin de 7, par exemple un tampon phosphate rendu isotonique par addition de chlorure de sodium.

L'expression "un anticorps monoclonal fluorescent" signifie que l'anticorps a été rendu fluorescent par un fluorochrome approprié tel que l'isocyanate de fluorescéine.

L'expression "un anticorps monoclonal marqué par une sonde radioisotopique" signifie que l'anticorps monoclonal porte, soit sur un élément de sa structure, par exemple les résidus de tyrosine constitutifs, soit sur un radical approprié qui lui a été fixé, un isotope radioactif permettant de le doser par comptage de la radioactivité qui lui est associée.

L'expression "un anticorps monoclonal marqué par une sonde enzymatique" signifie que l'anticorps monoclonal est couplé à une enzyme qui, associée à l'emploi de réactifs appropriés, permet une mesure quantitative de cet anticorps monoclonal.

Le substrat et les réactifs sont choisis de sorte que le produit final de la réaction ou de la séquence de réactions provoquée par l'enzyme et mettant en oeuvre ces substances soit :
- ou bien une substance colorée ou fluorescente qui diffuse dans le milieu liquide environnant les cellules et qui fait l'objet, soit de la mesure finale spectrophotométrique ou fluorimétrique, respectivement, soit d'une évaluation à l'oeil, éventuellement par comparaison à une gamme de teintes étalonnées,
- ou bien une substance colorée insoluble qui se dépose sur les cellules et le support sur lequel elles sont fixées et qui peut faire l'objet, soit d'une mesure photométrique par réflexion, soit d'une évaluation à l'oeil, éventuellement par comparaison à une gamme de teintes étalonnées.

Lorsque l'on utilise un anticorps rendu fluorescent, la fluorescence associée aux cellules est lue directement sur un appareil approprié.

Lorsque l'on utilise une sonde radioisotopique, comme par exemple l'iode 125, la radioactivité associée aux cellules est comptée dans un compteur gamma selon toute modalité appropriée, par exemple après solubilisation des cellules par une solution alcaline (par exemple une solution de soude) et récupération de la solution contenant la radioactivité à l'aide d'un tampon absorbant.

Lorsque l'on utilise une sonde enzymatique sur l'anticorps monoclonal, l'apparition d'un produit coloré ou fluorescent est obtenue en ajoutant au support solide sur lequel a été fixée la population cellulaire portant l'antigène à doser, une solution contenant le substrat de l'enzyme et un ou plusieurs réactifs auxiliaires permettant d'obtenir finalement comme produit de réaction, soit un produit coloré soluble dans le milieu, soit un produit coloré insoluble, soit un produit fluorescent soluble, comme cela a été expliqué précédemment. On mesure ensuite le signal lumineux provenant des échantillons ainsi traités, à l'aide de l'appareillage adapté à chaque cas : photomètre en transmission, ou en réflexion ou fluorimètre respectivement. Alternativement, on peut aussi évaluer à l'oeil la coloration obtenue, en s'aidant éventuellement d'une gamme de solutions colorées étalonnées.

En utilisant comme sonde enzymatique la phosphatase alcaline, le couplage de cette enzyme avec l'anticorps monoclonal est effectué selon la méthode proposée par Boehringer Mannheim-Biochemica. Les substrats préférentiels de cette enzyme sont le paranitrophénylphosphate pour une lecture finale spectrophotométrique ou le méthyl-4-umbelliféryl phosphate pour une lecture fluorométrique ou le bromo-5 chloro-4 indolyl-3 phosphate pour obtenir un produit de réaction coloré insoluble. On peut de même utiliser comme sonde enzymatique la β-galactosidase dont les substrats préférentiels seront alors l'orthonitrophényl β-D-galactopyranoside ou le méthyl-4 umbelliféryl β-D-galactopyranoside.

Préférentiellement, on peut coupler les anticorps monoclonaux à la peroxydase. Dans ce cas, le procédé de couplage est dérivé de celui décrit par M.B. WILSON et P.K. NAKANE dans Immunofluorescence and Related Staining Techniques, W. Knapp, K. Kolubar, G. Wicks ed. Elsevier/North Holland, Amsterdam, 1978, p. 215-224. Les modifications introduites par rapport au protocole initial de préparation du conjugué enzymatique portent sur les points suivants :
- rapport molaire peroxydase/anticorps égal à 3, contre 2 dans le protocole,
- oxydation moins poussée des motifs glucidiques de la peroxydase par diminution de 33% de la concentration proposée en periodate de sodium.

Les réactifs utilisés pour révéler la peroxydase conjuguée aux anticorps monoclonaux contiennent de l'eau oxygénée, substrat de l'enzyme, et un chromogène approprié par exemple de l'orthophénylènediamine ou l'acide azino-2-2' bis (éthyl-3 thiazoline sulfonique-6) ou ABTS pour obtenir un produit final de réaction coloré et soluble dans le milieu ou bien la diamino-3,3' benzidine ou l'amino-3 éthyl-9 carbazole ou le chloro-4 α-naphtol pour obtenir un produit final de réaction insoluble, ou bien l'acide parahydroxyphényl propionique pour obtenir un produit final de réaction fluorescent soluble dans le milieu.

Un autre mode de réalisation de l'invention est l'utilisation d'anticorps monoclonaux couplés à l'acétylcholinestérase.

L'acétylcholinestérase est couplée à l'anticorps en utilisant préférentiellement un procédé dérivé de celui décrit dans le brevet français n° 2 550 799 ou un procédé qui comporte schématiquement la préparation de fragments de l'anticorps par une technique connue, la modification de l'enzyme par réaction avec un agent hétérobifonctionnel approprié et enfin le couplage des produits ainsi obtenus. D'autres procédés connus de construction de conjugués immunoenzymatiques peuvent aussi être utilisés dans ce cas.

La révélation de l'activité enzymatique spécifiquement liée à l'antigène cellulaire reconnu par le conjugué à l'acétylcholinestérase est réalisée de préférence selon la technique bien connue qui emploie l'acétylthiocholine comme substrat de l'enzyme et le réactif d'Ellman, ou acide dithio-5-5' nitro-2 benzoïque comme chromogène, selon toute variante adaptée au cas examiné, par exemple celle décrite par Pradelles et al., Anal. Chem., 1985, 57, 1170-1173.

Les chromogènes cités sont utilisés tels quels ou sous forme de sels solubles dans l'eau.

Selon la présente invention, les baguettes (baguette de mesure et baguette(s) de référence) sont des supports solides qui ont une taille adaptée à celle du récipient contenant les particules magnétiques. En effet, les baguettes sont destinées à être partiellement plongées dans ledit récipient puis retirées aisément. Ainsi, la baguette de mesure pourvue d'un aimant terminal permet de capter toutes les particules magnétiques contenues dans le deuxième récipient de la trousse de dosage.

Comme baguette de mesure, on peut par exemple choisir une spatule plate d'une dizaine de centimètres de long, d'environ 1 cm de large et d'un à deux millimètres d'épaisseur, adaptée à la dimension des tubes à hémolyse.

La partie magnétique peut alors recouvrir une surface d'environ 1 cm² sur chaque face de la spatule. On peut par exemple découper des pastilles de ruban magnétique Flexor 15 ® , commercialisé par la Société Arelec, de dimensions appropriées et les coller sur chaque face de la baguette. On peut également mouler un aimant à l'intérieur de la baguette dans sa partie terminale.

La ou les baguettes de référence ont de préférence la même longueur que la baguette de mesure, mais elles sont plus étroites, par exemple, leur section est circulaire (environ 2 mm de diamètre) ; de plus, les baguettes de mesure et de référence peuvent être solidaires à une extrémité, ce qui permet de les manipuler ensemble pour les opérations de capture, de marquage et de lavage. Elles peuvent être aisément séparées avant la mesure proprement dite, effectuée sur chaque baguette, à savoir la révélation de l'activité enzymatique fixée sur l'anticorps dans le cas d'un marquage immunoenzymatique ou le comptage de la radioactivité dans le cas d'un marquage radioisotopique.

Sur la partie terminale de la ou des baguettes de référence, on a fixé par exemple par adsorption physique ou liaison covalente, une quantité déterminée d'antigène CD15 purifié ou non. On peut également fixer une quantité déterminée d'anticorps anti-espèce, ladite espèce étant l'espèce animale de laquelle proviennent les anticorps monoclonaux anti-CD15 utilisés, par exemple des anticorps anti-immunoglobine de souris lorsque l'anticorps anti-CD15 est d'origine murine.

En effet, selon la présente invention, la baguette de référence est destinée à capter une quantité connue et fixée à l'avance de l'anticorps anti-CD15 marqué par une sonde enzymatique ou radioisotopique, présent dans le réactif de dilution et de marquage.

La quantité d'antigène CD15 ou d'anticorps anti-espèce, fixée sur la baguette de référence, est déterminée à l'avance de telle sorte que le signal mesurable à la fin du procédé selon l'invention corresponde à une valeur seuil du nombre de granulocytes dans l'échantillon soumis à l'analyse.

Lorsque l'on utilise plusieurs baguettes de référence, chacune est recouverte d'une quantité différente d'antigène CD15 ou d'anticorps anti-espèce, de telle sorte que le signal mesurable à la fin du procédé pour chaque baguette de référence corresponde à une valeur différente du nombre de granulocytes.

Lorsque l'on effectue le dénombrement des granulocytes sur un échantillon de sang, on peut choisir une valeur seuil qui correspond par exemple à la valeur minimale du nombre moyen de granulocytes comptés chez des sujets sains : environ 2000 cellules par mm³ de sang. On peut également choisir une valeur inférieure qui correspond à une granulopénie plus sévère : entre 500 et 1000 cellules par mm³ par exemple.

L'accès des cellules à la partie aimantée de la baguette de mesure ne doit pas être empêché ; pour cela la ou les baguettes de référence sont plus étroites que la baguette de mesure ; de plus, on évite que les baguettes puissent se toucher en choisissant une géométrie appropriée pour chacune d'elles. Par exemple la présence, sur l'une des baguettes, d'un renflement faisant saillie vers l'autre baguette, permet de maintenir ces baguettes à une distance donnée par exemple d'un à deux millimètres.

Le dispositif permettant de solidariser temporairement les baguettes est situé, soit sur la baguette de mesure, à l'opposé de sa partie aimantée, soit sur la baguette de référence, à l'opposé de sa partie portant les anticorps anti-espèce ou l'antigène CD15.

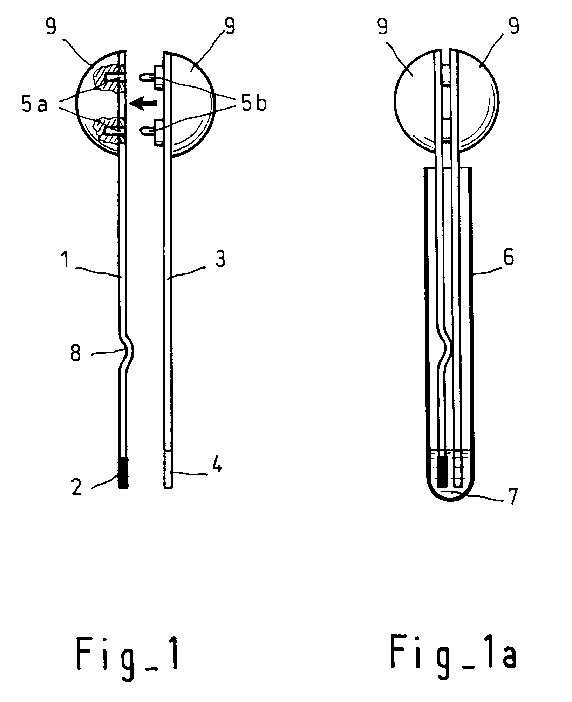

Un mode particulier de réalisation des deux baguettes est représenté par les figures 1 et 1 a, sur lesquelles (1) représente la baguette de mesure munie à sa partie inférieure de la partie aimantée (2) ; (3) représente la baguette de référence comportant à sa partie inférieure une zone (4) revêtue d'anticorps anti-immunoglobulines de souris.

Les baguettes (1) et (3) comportent des moyens conjugués mâle (5b) et femelle (5a) pour leur fixation mutuelle démontable.

La figure 1a représente les baguettes (1) et (3) associées lors de l'opération de dosage, lesdites baguettes étant insérées dans un récipient (6), contenant le milieu réactionnel ou le liquide de lavage (7), tel qu'un tube. De plus, l'une des baguettes comporte un renflement (8) faisant saillie vers l'autre baguette. Les baguettes peuvent être munies de moyens de préhension (9), tels que des demi-sphères.

Selon un mode de réalisation de l'invention, on peut utiliser comme dispositif de lavage différents récipients contenant le liquide de lavage dans lesquels on trempe la baguette de mesure et, le cas échéant, la (ou les) baguette(s) de référence à laquelle elle est (ou auxquelles elles sont) solidarisée(s) . Les récipients choisis pour le lavage peuvent avoir une dimension semblable à celle du deuxième récipient de la trousse de dosage contenant les particules magnétiques, et le lavage est effectué par trempage de la baguette de mesure et , le cas échéant, de la ou des baguettes de référence, successivement dans chacun des récipients contenant le liquide de lavage.

Selon un autre mode de réalisation, on peut utiliser comme dispositif de lavage, un récipient contenant la quantité totale de liquide de lavage nécessaire pour un dosage, ledit dispositif étant muni d'un système d'écoulement ; il peut de plus être adaptable sur le récipient contenant les particules magnétiques (deuxième récipient), dans lequel l'écoulement du liquide de lavage s'opère. Le récipient contenant les particules magnétiques comprend alors lui aussi un dispositif d'écoulement. L'écoulement du liquide de lavage permet un rinçage de la baguette de mesure et, le cas échéant, de la (des) baguette(s) de référence, placées dans le deuxième récipient de la trousse de dosage.

La présente invention a également pour objet un procédé de dénombrement rapide des granulocytes, utilisant la trousse selon l'invention et caractérisé en ce qu'il comprend :
a) le mélange d'une quantité déterminée d'un échantillon de sang humain ou de liquide biologique humain à une quantité fixe de réactif de dilution et de marquage des granulocytes contenant l'anticorps spécifique, ledit anticorps étant marqué de façon usuelle ;
b) le transfert de l'échantillon ainsi obtenu et son agitation dans un récipient contenant les particules magnétiques sur lesquelles est fixé le même anticorps spécifique non marqué ;
c) l'introduction dans le récipient de la baguette de mesure pourvue d'une partie terminale aimantée et, éventuellement, d'au moins une baguette de référence, ladite baguette de référence étant recouverte sur sa partie terminale d'une quantité déterminée soit d'anticorps polyclonaux anti-espèce, ladite espèce étant l'espèce animale de laquelle provient l'anticorps spécifique, soit d'antigène spécifique ;
d) après un temps de réaction, le retrait de la baguette de mesure et, le cas échéant, de la ou des baguettes de référence et le lavage de la baguette de mesure et, le cas échéant, de la ou des baguettes de référence ;
e) le dénombrement proprement dit des granulocytes.

Selon un autre mode de réalisation du procédé selon l'invention, on peut intervertir les étapes a) et b).

Le temps de réaction mentionné à l'étape d) est compris entre 1 et 5 minutes.

A l'étape d), on peut effectuer le lavage, soit par un courant de liquide de lavage, soit par trempages successifs dans les récipients contenant le liquide de lavage , pendant 5 à 10 secondes dans chaque récipient.

Selon un mode de réalisation particulier de l'invention, l'anticorps spécifique utilisé est un anticorps monoclonal anti-CD 15. La fixation de celui-ci sur les particules magnétiques est réalisée par adsorption physique ou par liaison covalente. Lorsque l'anticorps spécifique est marqué par une sonde enzymatique, le procédé selon l'invention comprend également le traitement de la baguette de mesure par le substrat de l'enzyme et, éventuellement, un ou des réactifs auxiliaires appropriés et, le cas échéant, le même traitement appliqué à la ou aux baguettes de référence.

La mesure proprement dite des granulocytes fixés sur la baguette de mesure dépend du mode de marquage de l'antigène spécifique. Par exemple, elle s'effectue par comptage de la radioactivité fixée ou, alternativement, par mesure photométrique en transmission ou en réflexion, ou par mesure de l'émission de fluorescence ou par évaluation à l'oeil éventuellement par comparaison à une gamme de signaux étalonnés ; le cas échéant, la mesure du signal photométrique ou de radioactivité provenant de la ou des baguettes de référence s'effectue de la même manière, puis on réalise, la comparaison entre les signaux photométriques ou de radioactivité provenant de la ou des baguettes de référence et de la baguette de mesure.

Selon un mode particulier de réalisation de l'invention, la ou les baguettes de référence utilisées sont solidaires de la baguette de mesure. Dans ce cas, la désolidarisation des différentes baguettes intervient dans une étape d1), après l'étape d).

Selon la présente invention, on peut utiliser plusieurs baguettes, l'une dite baguette de mesure, la ou les autres dites baguettes de référence, ces dernières permettant de connaître les résultats pour une ou plusieurs valeurs du nombre de granulocytes. Ainsi, lorsque l'on souhaite effectuer un dénombrement précis des granulocytes présents dans un échantillon, on mesure à la fin du procédé l'absorbance due aux cellules marquées portées par la baguette de mesure, les valeurs des absorbances mesurées provenant de la ou des baguettes de référence servant d'étalon interne. Lorsque l'on souhaite comparer le nombre de granulocytes de l'échantillon à doser avec la ou les valeurs seuils choisies, une comparaison visuelle de la coloration obtenue pour chacune des baguettes peut suffire.

De façon classique, on révèle par exemple l'activité de la peroxydase pendant 20 minutes, tandis que l'on révèle l'activité de la phosphatase alcaline pendant environ 2 heures (demande de brevet EP 311 492). On mesure ensuite l'intensité de la coloration obtenue (absorbance) à l'aide d'un spectrophotomètre.

Selon la présente invention, il a été trouvé qu'un temps de révélation de 2 minutes pour la peroxydase est suffisant pour l'observation visuelle des différences d'absorbances entre la baguette de mesure et la (les) baguette(s) de référence. Il est également suffisant pour mesurer l'absorbance des cellules portées par la baguette de mesure. De même, pour toute enzyme utilisée pour le marquage, on peut raccourcir substantiellement le temps de révélation proposé classiquement pour les dosages immunométriques.

Selon un mode de réalisation préférentiel de l'invention, le dénombrement des granulocytes d'un échantillon de sang est effectué par le procédé selon l'invention en utilisant la trousse selon l'invention, contenant à la fois la baguette de mesure et la baguette de référence et dans laquelle l'anticorps monoclonal anti-CD15 est une immunoglobuline d'isotype M marquée à la peroxydase. Dans ce cas, les différentes étapes du procédé sont effectuées selon les temps indiqués approximativement ci-après :
étape a) : 15 secondes
étape b) : 15 secondes
étape d) : 2 minutes 30 secondes

Lorsque l'anticorps anti-CD 15 est marqué par une sonde enzymatique, l'étape de traitement de la baguette de mesure par le substrat de l'enzyme est inférieure à 2 minutes, de même pour le traitement éventuel de la ou des baguettes de référence.

Ainsi, la durée totale d'exécution est inférieure à 6 minutes.

Dans les exemples qui vont suivre, on utilisera indifféremment les termes suivants ou leurs abréviations :
- BSA :: albumine de sérum bovin, ou sérum albumine bovine
- PBS :: tampon phosphate salin à pH 7,4
- IgG :: immunoglobuline G
- IgM:: immunoglobuline M
- cpm :: coups par minute
- dpm :: désintégrations par minute
- EDTA :: acide éthylènediamine tétraacétique
- POD :: peroxydase

### EXEMPLE 1

Mesure des granulocytes à partir d'échantillons de sang humain.

### A) Préparation de la trousse de dosage.

### a) Préparation des particules magnétiques.

On procède comme dans le brevet européen 104 101 et en ajoutant des oxydes magnétiques.

On prépare une solution contenant :
- 3,8 ml d'acide méthacrylique
- 121,9 ml de solution aqueuse de N-,N'- méthylène bis acrylamide à 20 g par litre
- 88,1 ml d'acroléine
- 93,2 ml d'hydroxyéthylméthacrylate
- 193 ml d'eau

Ainsi cette solution, appelée solution mère, contient 37,5% en volume des monomères.

On utilise un tensio-actif fluoré commercialisé par Atochem : le Forafac ® 1157, dilué à 18,8 g par litre.

Comme oxyde magnétique, on utilise EGM 805 ®, commercialisé par Ferrofluidics.

Dans un tube à sceller de 18,8 ml, on mélange:
- 2,5 ml de solution mère
- 0,1 ml de solution de tensioactif
- 1 ml d'EGM 805 ®
- la quantité nécessaire d'eau

Après barbotage d'un courant d'azote, les milieux réactionnels sont congelés dans l'azote liquide et les ampoules sont scellées sous vide.

La polymérisation est provoquée par irradiation par les rayons γ d'une bombe au Cobalt (⁶⁰Co) pendant 3 heures à 60 krad/heure.

Le diamètre des particules magnétiques obtenues est compris entre 1 et 10 µm.

### b) Préparation des tubes contenant les particules magnétiques.

On utilise soit des particules préparées à l'étape a), soit des particules de latex Estapor ® , de référence M1.070/40, commercialisées par Rhône-Poulenc, dont le diamètre moyen est de 0,8 µm. Ces particules sont couplées à un anticorps de spécificité CD15. L'anticorps choisi est l'anticorps d'isotype M, SMY 15a, commercialisé par Biosys. Le couplage des particules de latex à l'anticorps est réalisé selon le mode opératoire décrit ci-après.

Dans un tube en verre, on place 0,1 ml d'une suspension à 10% de particules de latex. On ajoute 1 mg de solution d'anticorps dans 1 ml de tampon PBS, puis on agite pendant 30 minutes à 56°C. Après une nuit à 4°C, on centrifuge (15.10³ tours par minute, 4°C) pendant 30 à 60 minutes, puis on lave par 1 ml de tampon PBS et centrifuge à nouveau. Enfin on sature par 1 ml de tampon PBS contenant 0,2% de BSA.

Les particules ainsi obtenues sont placées dans 1 ml de tampon phosphate salin contenant 0,2% d'albumine bovine. On introduit 25 µl de ce mélange dans un tube à hémolyse de 5 ml de volume. Les tubes ainsi préparés sont lyophilisés, bouchés puis stockés à + 4°C dans un sachet plastique scellé.

### c) Préparation des dispositifs contenant la solution de conjugué anticorps monocional-peroxydase.

On utilise la peroxydase (POD) commercialisée par Boehringer Mannheim Biochemica (référence 814 393).

Le procédé de couplage entre l'anticorps et la peroxydase est celui décrit par M.B. WILSON et P.K. NAKANE dans Immunofluorescence and Related Staining Techniques (W. Knapp, K. Kolubar, G. Wicks ed. Elsevier/North Holland, Amsterdam, 1978, p. 215-224), excepté que l'on met en oeuvre pour l'oxydation de la peroxydase, 1,5 mg de POD dans 0,36 ml d'eau distillée et que l'on ajoute 50 µl d'une solution 0,2 M de periodate de sodium. Le produit ainsi obtenu est couplé à 2 mg d'IgM anti-CD15 contenus dans 500 µl de tampon carbonate. Après traitement par le borohydrure de sodium et dialyse contre le PBS, le conjugué IgM-POD est stérilisé par filtration sur membrane de 0,22 µm.

Le conjugué anticorps SMY-15a-peroxydase est conditionné en doses unitaires de 400 µl de réactif prêt à l'emploi, à la concentration de 2 µg d'anticorps par ml d'un mélange volume à volume de tampon PBS et de sérum de veau foetal. Le conditionnement peut être effectué soit dans des tubes à hémolyse de 5 ml, soit dans des dispositifs Unopette®, commercialisés par Becton-Dickinson, référence 5856.

### d) Préparation du réactif de révélation.

On dissout 810 mg de dichlorhydrate d'orthophenylène diamine dans 25 ml de tampon PBS et on prépare des tubes à hémolyse de 5 ml contenant chacun 50 µl de cette solution. Les tubes sont lyophilisés, bouchés et gardés à + 4°C dans un sachet plastique scellé. Juste avant l'utilisation, on ajoute dans chaque tube 600 µl d'une solution de révélation : tampon citrate et eau oxygénée (substrat de l'enzyme), commercialisée par Pasteur Sanofi Diagnostics

### e) Préparation des baguettes de mesure.

On utilise des spatules de plastique blanc de 10 cm de long, commercialisées par Safaa. A l'extrémité de chaque spatule, on fixe sur chaque face une pastille de 8 mm de diamètre de ruban magnétique autocollant, commercialisé par Arelec (référence : Flexor 15, 15/10^{e}). Les spatules sont lavées pendant 1 minute par de l'eau distillée et un détergent Manisoft ® , commercialisé par Paragem (France), rincées à l'eau distillée puis séchées et gardées dans un sachet en aluminium.

### f) Préparation des baguettes de référence.

On utilise des spatules de plastique de 10 cm de long et de deux millimètres de diamètre, commercialisées par Somater. Des anticorps anti-immunoglobuline de souris sont adsorbés sur une extrémité de la baguette en procédant de la façon suivante : l'extrémité de la baguette (5 mm de hauteur) est plongée pendant 12 heures dans une solution à 4°C, contenant 4µg/ml d'anticorps en solution dans un tampon PBS ; on plonge ensuite la baguette pendant 10 minutes dans une solution d'albumine bovine à 0,2% dans un tampon PBS ; enfin on sèche la baguette.

### g) Préparation des tubes de lavage.

Dans plusieurs tubes à hémolyse de 5 ml, on introduit 4 ml de tampon PBS, puis on ferme les tubes qui sont ainsi prêts à l'emploi.

### B) Dosage des échantillons sanguins.

### a) Marquage et capture des granulocytes.

Dans un tube préparé à l'étape A c), contenant le conjugué anticorps-POD, on dépose 44 µl de sang prélevé sur anticoagulant EDTA et on mélange pendant 5 secondes. Le sang peut être, soit du sang veineux prélevé à l'aide d'une seringue équipée d'une aiguille, soit du sang capillaire prélevé à l'extrémité du doigt, à l'aide d'un piqueur, dispositif commercialisé par Becton-Dickinson : Microtainer ® , référence 6357.

Lorsque l'on utilise le dispositif Unopette ® , contenant la solution du conjugué anticorps-POD, on utilise le tube capillaire proposé par le fabricant (Becton-Dickinson) pour introduire 44 µl de sang.

Un tube bouché contenant les particules magnétiques, préparé à l'étape A b) est ouvert et on y dépose rapidement le mélange de sang et de conjugué anticorps-POD, préparé précédemment, soit un volume total de 444µl. On agite pendant 15 secondes puis on introduit dans le tube la baguette de mesure préparée à l'étape A e) et la baguette de référence préparée à l'étape A f) et on laisse les baguettes pendant 2 minutes 30 dans le tube.

L'opération est réalisée avec 2 x 8 échantillons sanguins.

### b) Lavage et révélation de l'activité enzymatique.

Les baguettes sont retirées du tube de marquage puis lavées par passage et agitation de haut en bas dans 4 tubes de lavage successifs, à raison de 5 secondes par tube, soit pendant 20 secondes au total.

Les baguettes peuvent également être lavées par un courant de liquide de lavage pendant 20 secondes. Chaque baguette est ensuite plongée dans le tube de révélation contenant l'orthophenylène diamine, préparé à l'étape A d), dans lequel on ajoute 600 µl de solution de révélation.

### c) Résultats

Après 2 minutes, on constate que la coloration jaune du milieu de révélation provenant de la baguette de mesure d'un échantillon analysé, est plus intense que celle provenant de la baguette de référence.

De plus, l'absorbance du milieu de révélation est mesurée sur 200 µl de solution à l'aide d'un spectrophotomètre calibré à la longueur d'onde de 450 nm.

La mesure a été réalisée sur 2 x 8 échantillons sanguins provenant de donneurs sains, chaque donneur ayant subi 2 prélèvements : sang veineux et sang capillaire.

Par ailleurs, on a mesuré pour chaque échantillon de sang veineux à l'aide d'appareils automatiques, le nombre de granulocytes contenu dans 1 mm³ de sang. Cette mesure est effectuée par comptage automatique des cellules avec un appareil Ortho ELT 8, commercialisé par Diagnostic System. On utilise en outre un appareil Hematrak ® (Geometric Data) pour la détermination des pourcentages des sous-populations leucocytaires.

Les résultats sont rapportés dans le tableau 1 ci-après.

**Tableau 1**

| Echantillon | Absorbance à 450 nm | | Nombre de granulocytes par mm³ de sang veineux |
|---|---|---|---|
| | Sang veineux | Sang capillaire | |
| témoin sans cellules | 0,025 | 0,025 | 0 |
| 1 | 0,253 | 0,315 | 3705 |
| 2 | 0,362 | 0,301 | 4408 |
| 3 | 0,239 | 0,194 | 3300 |
| 4 | 0,261 | 0,256 | 3776 |
| 5 | 0,179 | 0,195 | 1908 |
| 6 | 0,198 | 0,147 | 2000 |
| 7 | 0,274 | 0,203 | 3300 |
| 8 | 0,363 | 0,357 | 5994 |
| référence | | 0,130 | 1700 |

Il apparait dans le tableau 1 que les résultats mesurés sont similaires pour le sang veineux et le sang capillaire. De plus, on a calculé la valeur r des coefficients de corrélation entre l'absorbance mesurée pour chaque échantillon et le nombre de granulocytes comptés pour le même échantillon. Ce coefficient est r=0,955 pour les échantillons de sang veineux ; il est de r=0,943 pour les échantillons de sang capillaire.

### EXEMPLE 2

Efficacité de la méthode pour la capture des granulocytes dans le sang total.

Pour 5 échantillons sanguins prélevés sur des sujets sains, on a mesuré, par comptage automatique, tout d'abord le nombre de granulocytes présents dans 1 mm^{3,} puis on a effectué la même mesure après avoir pratiqué la méthode de capture par des particules magnétiques, telle que décrite à l'exemple 1. Pour chaque échantillon, le pourcentage de granulocytes capturés est obtenu par différence entre le nombre de granulocytes comptés avant et après la capture cellulaire à l'aide des particules magnétiques.

Dans ces essais, les particules magnétiques et les granulocytes ont été séparés du milieu réactionnel, soit par une baguette aimantée selon le mode opératoire décrit à l'exemple 1, soit au moyen d'un aimant fixe situé à l'extérieur des tubes et constitué par un barreau magnétique.

Les résultats obtenus sont rapportés dans le tableau 2 ci-dessous.

**Tableau 2**

| Echantillon | % des granulocytes capturés par particules magnétiques | | Nombre de granulocytes par mm³ de sang |
|---|---|---|---|
| | Baguette aimantée | Aimant fixe | |
| 1 | 97,2% | 96,9% | 1890 |
| 2 | 94,1% | 98,9% | 3016 |
| 3 | 98,5% | 99,5% | 3760 |
| 4 | 97,3% | 97,5% | 3060 |
| 5 | 99,4% | 97,7% | 6063 |

L'observation des résultats exprimés dans le tableau 2, montre que le pourcentage de granulocytes capturés par la technique selon l'invention est élevé (97 à 100%), quel que soit le nombre de granulocytes présents dans l'échantillon sanguin et quel que soit le type d'aimant choisi pour la capture.

### EXEMPLE 3

### Dosage des granulocytes en milieu urinaire infecté.

Dans un tube contenant les particules magnétiques, préparé à l'exemple 1, étape A b), on introduit 40 µl d'urine infectée et 400 µl de solution de conjugué anticorps anti-CD15 marqué à la peroxydase, tel que préparé à l'exemple 1, étape A c). Après 30 secondes d'agitation, on introduit une baguette de mesure préparée à l'exemple 1, étape A e) pendant 2 minutes 30. Ladite baguette est ensuite lavée par trempages successifs dans 4 tubes de 4 ml de PBS, en agitant la baguette pendant 5 secondes dans chaque tube. Enfin, la baguette est plongée dans 600 µl de réactif de révélation de la peroxydase, préparé à l'exemple 1, étape A d). Après 2 minutes, l'absorbance est mesurée à l'aide d'un spectrophotomètre à la longueur d'onde de 450 nm. On compare avec les résultats observés dans les mêmes conditions pour un échantillon d'urine non infectée. Par ailleurs, on mesure par un compteur de cellules le nombre de granulocytes contenus dans l'échantillon d'urine infectée.

Les résultats sont rapportés dans le tableau 3 ci-dessous:

**Tableau 3**

| Echantillon | Absorbance | Nombre de granulocytes/ml |
|---|---|---|
| urine infectée | 0.488 ± 0,073 | 120.10³ |
| urine non infectée | 0,012 | env.0 |

### EXEMPLE 4

### Dénombrement des granulocytes dans divers échantillons sanguins.

On utilise la trousse de dosage selon l'invention comportant une baguette de mesure et une baguette de référence telle que préparée à l'exemple 1 et l'on mesure les résultats obtenus pour 8 échantillons sanguins par spectrophotométrie à 450 nm. A titre de comparaison, on mesure à l'aide d'un compteur le nombre de granulocytes par mm³ de sang. Les résultats sont reportés dans le tableau 4.

**Tableau 4**

| Echantillon | Absorbance à 450 nm | | Nombre de granulocytes par mm³ de sang |
|---|---|---|---|
| | Baguette de mesure | Baguette de référence | |
| 1 | 0,195 | 0,130 | 1 584 |
| 2 | 0,280 | 0,136 | 2 745 |
| 3 | 0,272 | 0,127 | 2 880 |
| 4 | 0,266 | 0,141 | 3 190 |
| 5 | 0,310 | 0,128 | 3 192 |
| 6 | 0,290 | 0,110 | 3 591 |
| 7 | 0,307 | 0,118 | 3 650 |
| 8 | 0,408 | 0,142 | 5 022 |

A l'oeil nu, on observe pour chaque échantillon une coloration jaune de la baguette de mesure, plus intense que la coloration de la baguette de référence.

## Revendications

1. Trousse pour le dénombrement des granulocytes du sang humain ou de tout autre liquide biologique humain, comprenant comme composants :
a) un premier récipient contenant une quantité déterminée de réactif de dilution et de marquage des granulocytes contenant un anticorps spécifique marqué d'une façon usuelle ;
b) un deuxième récipient contenant des particules magnétiques sur lesquelles est fixé le même anticorps spécifique non marqué ;
c) une baguette (1), dite baguette de mesure, pourvue d'une partie terminale aimantée (2).

2. Trousse selon la revendication 1, caractérisée en ce qu'elle comprend en outre :
d) un dispositif permettant de prélever une quantité déterminée de sang ou de liquide biologique ;
e) un dispositif contenant du liquide de lavage ;
f) des moyens pour révéler le marqueur de l'anticorps spécifique ;
g) au moins une baguette (3), dite baguette de référence, ladite baguette étant recouverte sur sa partie terminale (4) d'une quantité déterminée, différente pour chacune, soit d'anticorps polyclonaux anti-espèce, ladite espèce étant l'espèce animale de laquelle provient l'anticorps spécifique, soit d'antigène spécifique.

3. Trousse selon la revendication 2, caractérisée en ce que la ou lesdites baguettes de référence comportent des moyens démontables (5b) de fixation avec la baguette de mesure.

4. Trousse selon l'une quelconque des revendications 1 à 3, caractérisée en ce que les particules magnétiques ont une dimension comprise entre 0,1 et 20 µm.

5. Trousse selon la revendication 4 dans laquelle les particules magnétiques ont une dimension comprise entre 0,5 et 4 µm.

6. Trousse selon l'une quelconque des revendications 1 à 5, caractérisée en ce que l'anticorps spécifique est marqué par un agent fluorescent, une sonde enzymatique ou une sonde radioisotopique.

7. Trousse selon l'une quelconque des revendications 2 à 6, caractérisée en ce que l'anticorps spécifique est marqué par une sonde enzymatique, les moyens f) sont constitués par un ou plusieurs récipients contenant le substrat de l'enzyme et, le cas échéant, un ou plusieurs réactifs nécessaires pour mesurer l'activité de l'enzyme.

8. Trousse selon l'une quelconque des revendications 1 à 7, caractérisé en ce que l'anticorps spécifique est un anticorps monoclonal anti-CD15.

9. Trousse selon la revendication 8, caractérisée en ce que l'anticorps monoclonal anti-CD15 est une immunoglobuline d'isotype IgM.

10. Procédé pour le dénombrement rapide de granulocytes, caractérisé en ce qu'il comprend :
a) le mélange d'une quantité déterminée d'un échantillon de sang humain ou de liquide biologique humain à une quantité fixe de réactif de dilution et de marquage des granulocytes contenant un anticorps spécifique, ledit anticorps étant marqué de façon usuelle ;
b) le transfert de l'échantillon ainsi obtenu et son agitation dans un récipient contenant des particules magnétiques sur lesquelles est fixé le même anticorps spécifique non marqué ;
c) l'introduction dans le récipient de la baguette de mesure (1) pourvue d'une partie terminale aimantée (2) et, éventuellement, d'au moins une baguette de référence (3), ladite baguette de référence étant recouverte sur sa partie terminale (4) d'une quantité déterminée, soit d'anticorps polyclonaux anti-espèce, ladite espèce étant l'espèce animale de laquelle provient l'anticorps spécifique, soit d'antigène spécifique ;
d) après un temps de réaction, le retrait de la baguette de mesure et, le cas échéant, de la ou des baguettes de référence et le lavage de la baguette de mesure, et le cas échéant, de la ou des baguettes de référence ;
e) le dénombrement proprement dit des granulocytes.

11. Procédé selon la revendication 10, caractérisé en ce que les étapes a) et b) sont interverties.

12. Procédé selon l'une des revendications 10 ou 11, caractérisé en ce que l'anticorps monoclonal spécifique est un anticorps monoclonal anti-CD15.

13. Procédé selon la revendication 12, caractérisé en ce que l'anticorps monoclonal anti-CD15 est une immunoglobuline d'isotype IgM.

14. Procédé selon l'une quelconque des revendications 10 à 13, caractérisé en ce que à l'étape d), le temps de réaction est compris entre une et cinq minutes.

## Patentansprüche

1. Kit zur quantitativen Bestimmung der Anzahl der Granulocyten des menschlichen Bluts oder einer beliebigen anderen biologischen Flüssigkeit menschlichen Ursprungs,
der folgende Bestandteile aufweist:
a) einen ersten Behälter, der eine festgelegte Menge eines Verdünnungs- und Markierungsreagens zur Verdünnung und Markierung von Granulocyten enthält, das einen in einer üblichen Weise markierten spezifischen Antikörper enthält;
b) einen zweiten Behälter, der magnetische Partikel enthält, auf denen der gleiche spezifische, aber nicht markierte Antikörper gebunden ist;
c) ein als Meßstäbchen bezeichnetes Stäbchen (1), das einen magnetischen Endteil (2) aufweist.

2. Kit nach Anspruch 1, dadurch gekennzeichnet, daß er ferner enthält:
d) eine Vorrichtung, welche die Entnahme einer festgelegten Menge Blut oder der biologischen Flüssigkeit erlaubt;
e) eine Vorrichtung, die Waschflüssigkeit enthält;
f) eine Einrichtung zur Bestimmung des Markers des spezifischen Antikörpers;
g) mindestens ein als Referenzstäbchen bezeichnetes Stäbchen (3), das auf seinem Endteil (4) mit einer festgelegten, fallabhängig unterschiedlichen Menge entweder gegen eine Species gerichteter polyklonaler Antikörper, wobei die Species die tierische Species ist, von welcher der polyklonale Antikörper stammt, oder eines spezifischen Antigens beschichtet ist.

3. Kit nach Anspruch 2, dadurch gekennzeichnet, daß das oder die Referenzstäbchen eine Einrichtung (5b) zur lösbaren Befestigung am Meßstäbchen aufweisen.

4. Kit nach einem der Ansprüche 1 bis 3, dadurch gekennzeichnet, daß die magnetischen Partikel eine Größe von 0,1 bis 20 µm aufweisen.

5. Kit nach Anspruch 4, wobei die magnetischen Partikel eine Größe von 0,5 bis 4 µm aufweisen.

6. Kit nach einem der Ansprüche 1 bis 5, dadurch gekennzeichnet, daß der spezifische Antikörper mit einem fluoreszierenden Mittel, einer Enzymsonde oder einer Radioisotopsonde markiert ist.

7. Kit nach einem der Ansprüche 2 bis 6, dadurch gekennzeichnet, daß der spezifische Antikörper mit einer Enzymsonde markiert ist und die Einrichtung f) aus einem oder mehreren Behältern besteht, die das Substrat des Enzyms sowie erforderlichenfalls ein oder mehrere Reagentien enthalten, die zur Messung der Enzymaktivität erforderlich sind.

8. Kit nach einem der Ansprüche 1 bis 7, dadurch gekennzeichnet, daß der spezifische Antikörper ein monoklonaler Anti-CD15-Antikörper ist.

9. Kit nach Anspruch 8, dadurch gekennzeichnet, daß der monoklonale Anti-CD15-Antikörper ein Immunglobulin vom IgM-Isotyp ist.

10. Verfahren zur raschen quantitativen Bestimmung der Anzahl von Granulocyten,
dadurch gekennzeichnet, daß es folgende Schritte umfaßt:
a) Mischen einer vorgegebenen Menge einer Probe von menschlichem Blut oder einer biologischen Flüssigkeit menschlichen Ursprungs mit einer festgelegten Menge Verdünnungs- und Markierungsreagens für Granulocyten, das einen spezifischen Antikörper enthält, der in einer üblichen Weise markiert ist;
b) Übertragen der so erhaltenen Probe in einen Behälter, der magnetische Partikel enthält, auf denen der gleiche spezifische, aber nicht markierte Antikörper gebunden ist, und Bewegen;
c) Einführen des mit einem magnetischen Endteil (2) versehenen Meßstäbchens (1) in den Behälter und ggf. auch Einführen mindestens eines Referenzstäbchens (3), das auf seinem Endteil(4) mit einer festgelegten Menge entweder gegen eine Species gerichteter polyklonaler Antikörper, wobei die Species die tierische Species ist, von welcher der polyklonale Antikörper stammt, oder eines spezifischen Antigens beschichtet ist;
d) Herausziehen des Meßstäbchens und ggf. des oder der Referenzstäbchen nach Ablauf einer Reaktionszeit und Waschen des Meßstäbchens sowie ggf. des oder der Referenzstäbchen;
e) eigentliche quantitative Ermittlung der Anzahl der Granulocyten.

11. Verfahren nach Anspruch 10, dadurch gekennzeichnet, daß die Stufen a) und b) vertauscht werden.

12. Verfahren nach Anspruch 10 oder 11, dadurch gekennzeichnet, daß der spezifische monoklonale Antikörper ein monoklonaler Anti-CD15-Antikörper ist.

13. Verfahren nach Anspruch 12, dadurch gekennzeichnet, daß der monoklonale Anti-CD15-Antikörper ein Immunglobulin vom IgM-Isotyp ist.

14. Verfahren nach einem der Ansprüche 10 bis 13, dadurch gekennzeichnet, daß in Stufe d) die Reaktionszeit 1 bis 5 min beträgt.

## Claims

1. A kit for counting the granulocytes in human blood or any other human biological fluid, comprising the following components:
a) a first receptacle containing a determined quantity of a granulocyte diluting and labeling reagent containing a specific antibody labeled in conventional manner;
b) a second receptacle containing magnetic particles to which the same, but unlabeled, specific antibody is bound; and
c) a rod (1), called a measuring rod, provided with a magnetized terminal part (2).

2. A kit according to claim 1 which further comprises:
d) a device for removing a determined quantity of blood or biological fluid;
e) a device containing washing liquid;
f) means for developing the marker of the specific antibody; and
g) at least one rod (3), called a reference rod, which is coated on its terminal part (4) with a determined quantity, different for each rod, either of anti-species polyclonal antibodies, said species being the animal species from which the specific antibody originates, or of specific antigen.

3. A kit according to claim 2 wherein said reference rod or rods comprise releasable attachement means (5b) for fixing to the measuring rod.

4. A kit according to any one of claims 1 to 3 wherein the magnetic particles have a size of between 0.1 and 20 µm.

5. A kit according to claim 4 in which the magnetic particles have a size of between 0.5 and 4 µm.

6. A kit according to any one of claims 1 to 5 wherein the specific antibody is labeled with a fluorescent agent, an enzymatic probe or a radioisotopic probe.

7. A kit according to any one of claims 2 to 6 wherein the specific antibody is labeled with an enzymatic probe and means f) consist of one or more receptacles containing the substrate for the enzyme and, if appropriate, one or more reagents necessary for measuring the activity of the enzyme.

8. A kit according to any one of claims 1 to 7 wherein the specific antibody is an anti-CDT15 monoclonal antibody.

9. A kit according to claim 8 wherein the anti- CD15 monoclonal antibody is an immunoglobulin of isotype IgM.

10. A process for the rapid counting of granulocytes, which comprises:
a) the mixing of a determined quantity of a sample of human blood or human biological fluid with a fixed quantity of granulocyte diluting and labeling reagent containing a specific antibody, said antibody being labeled in conventional manner;
b) the transfer of the resulting sample and the shaking thereof in a receptacle containing magnetic particles to which the same, but unlabeled, specific antibody is bound;
c) the introduction into the receptacle of the measuring rod (1) provided with a magnetized terminal part (2) and optionally at least one reference rod (3), said reference rod being coated on its terminal part (4) with a determined quantity either of anti-species polyclonal antibodies, said species being the animal species from which the specific antibody originates, or of specific antigen;
d) after a reaction time, the withdrawal of the measuring rod and, if appropriate, the reference rod or rods and the washing of the measuring rod and, if appropriate, the reference rod or rods; and e) the actual counting of the granulocytes.

11. A process according to claim 10 wherein steps a) and b) are interchanged.

12. A process according to claim 10 or claim 11 wherein the specific monoclonal antibody is an anti- CD15 monoclonal antibody.

13. A process according to claim 12 wherein the anti-CD15 monoclonal antibody is an immunoglobulin of isotype IgM.

14. A process according to any one of claims 10 to 13 wherein the reaction time in step d) is between one and five minutes.
